(19) Europäisches Patentamt
European Patent Office
Office européen des brevets

(11) EP 2 743 252 A1

(12) **EUROPEAN PATENT APPLICATION**
published in accordance with Art. 153(4) EPC

(43) Date of publication:
**18.06.2014 Bulletin 2014/25**

(21) Application number: **12815278.2**

(22) Date of filing: **09.07.2012**

(51) Int Cl.:
***C07C 211/38*** *(2006.01)*
***A61K 31/282*** *(2006.01)*
***A61P 35/00*** *(2006.01)*
***C07C 55/08*** *(2006.01)*
***C07F 15/00*** *(2006.01)*

(86) International application number:
**PCT/JP2012/067471**

(87) International publication number:
**WO 2013/011858 (24.01.2013 Gazette 2013/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.07.2011 JP 2011156527**

(71) Applicant: **Unitech Co., Ltd**
**Kashiwa-shi, Chiba 277-0005 (JP)**

(72) Inventors:
- **ARAI, Hisae**
**Kashiwa-shi**
**Chiba 277-0005 (JP)**
- **KONDO, Hisao**
**Kashiwa-shi**
**Chiba 277-0005 (JP)**
- **MASUDA, Norio**
**Kashiwa-shi**
**Chiba 277-0005 (JP)**

(74) Representative: **Rau, Schneck & Hübner**
**Patentanwälte Rechtsanwälte PartGmbB**
**Königstraße 2**
**90402 Nürnberg (DE)**

(54) **TETRAVALENT PLATINUM COMPLEX AND PHARMACEUTICAL COMPOSITION CONTAINING SAME**

(57) New platinum(IV) complexes which has a strong antitumor activity and has a feature that a side effect is relatively reduced is provided. The new complex is platinum(IV) complexes with cis,cis-spiro[4,4]nonane-1,6-diamine ligand and is platinum(IV) complexes with optically active cis,cis-spiro[4,4]nonane-1,6-diamine ligand. Especially, the new complex is dichloromalonato((1S,5S,6S)-spiro[4.4]nonane-1,6-diamine)platinum(IV).

EP 2 743 252 A1

**Description**

TECHNICAL FIELD

This invention relates to new platinum(IV) complexes and a pharmaceutical composition as an active ingredient, and particularly to a therapeutic agent for malignant tumors.

BACKGROUND ART

Recently, the malignant tumors come to hold the top of the cause of death. On the other hand, the various kinds of antitumor materials are developed. Among these, as to the platinum complex, the antitumor action is acknowledged conventionally, and such as cisplatin [I], carboplatin [II] and oxaliplatin [III] are developed, and used for medical treatment (for example, refer to Non-patent document No.1 - Non-patent document No.3). Besides, as the platinum complex which is effective to antitumor action, this applicant has Japanese patent registration No.4664424 (Patent document No.1) in relation to a new spiro[4,4]nonane-1,6-diamineplatinum(II) complex and the pharmaceutical composition which has an active ingredient thereof.

[Chemical Formula 1]

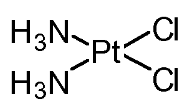

[I]
(1971)

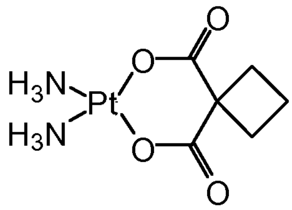

[II]
(1993)

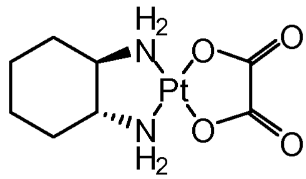

[III]
(2002)

PRIOR ART DOCUMENT

PATENT DOCUMENT

Patent document No.1: Japanese patent registration No.4664424
Patent document No.2: US Patent 4,140,707 (February 20, 1979)

NON-PATENT DOCUMENT

Non-patent document No.1: Nature, 1969, 222 385-386
Non-patent document No.2: Cancer Treat Reviews, 1985, 12 21-33
Non-patent document No.3: Cancer Letters, 1985, 27 135-143

SUMMARY OF THE INVENTION

PROBLEM TO BE SOLVED BY THE INVENTION

However, there were problems that the cisplatin has many side-effects such as nephrotoxicity, hematotoxicity, toxicity for digestive organs, and neurotoxicity. In this situation, the carboplatin was developed as the one which reduced the nephrotoxicity of the cisplatin and increased the water solubility, but the carboplatin was expensive. In addition, the antitumor action was not necessarily satisfactory. Although these have the antitumor activity, it is necessary to administer the predetermined required quantity corresponding to it to work the predefined antitumor activity. Therefore, there is a defect that these have the side-effects.

The object of this invention is to provide the new complex. The new complex is platinum(IV) complexes with cis,cis-spiro[4,4]nonane-1,6-diamine ligand and platinum(IV) complexes with optically active cis,cis-spiro[4,4]nonane-1,6-diamine ligand, and the complex has the stronger antitumor activity and has the effect with smaller doses, and thereby the side-effect is reduced relatively.

The object of this invention is to provide the new platinum(IV) complexes which has following feature. The new complex is dichloromalonato((1S,5S,6S)-spiro[4.4]nonane-1,6-diamine)platinum(IV) (G) which is described by the following formula (G) especially, and has the strong antitumor activity for malignant tumors, especially human lung cancer cell, human gastric cancer cell, human prostate cancer cell, human malignant melanoma cell, human bladder cancer cell, human lymphoma cell, human leukemia cell. And the side-effect is reduced relatively.

[Chemical Formula 2]

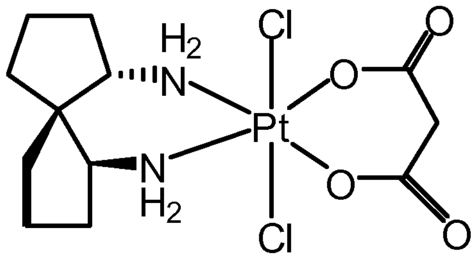

(G)

MEANS FOR SOLVING THE PROBLEMS

For achieving these objects, the therapeutic agent for the malignant tumor of this invention is platinum(IV) complexes with cis,cis-spiro[4,4]nonane-1,6-diamine (A) ligand which is represented by following general formula.

[Chemical Formula 3]

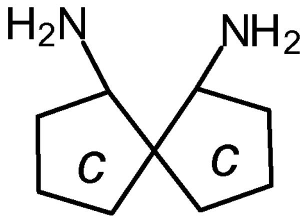

(A)

This invention is platinum(IV) complexes with optically active (1S,5S,6S)-spiro[4,4]nonane-1,6-diamine (B) ligand and platinum(IV) complexes with optically active (1R,5R,6R)-spiro[4,4]nonane-1,6-diamine (C) ligand.

[Chemical Formula 4]

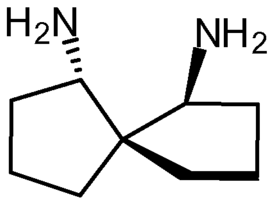

(B)

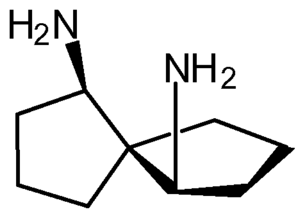

(C)

This invention is platinum(IV) complexes (D) with cis,cis-spiro[4,4]nonane-1,6-diamine (A) ligand which is represented by following general formula.

[Chemical Formula 5]

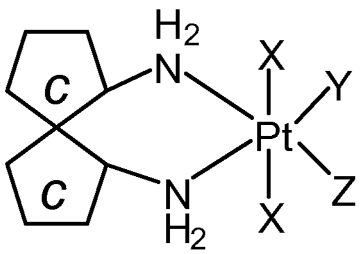

(D)

In the formula, X and Y, Z are same or different, and X and Y, Z represent halogen atoms or monovalent anionic group including acetate group respectively, or Y and Z cooperatively represent the divalent residue which is represented by the formula (b).

[Chemical Formula 6]

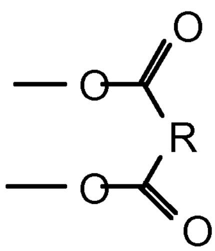

Formula (b)

In the formula, R represents the single bond or represents the straight-chain or branched-chain divalent hydrocarbon residue whose number of carbon atom is 1-6. The hydrocarbon residue may have the unsaturated bond, and the hydrocarbon residue may form the spiro structure.

This invention is platinum(IV) complexes (E) with optically active (1S,5S,6S)-spiro[4,4]nonane-1,6-diamine (B) ligand and platinum(IV) complexes (F) with optically active (1R,5R,6R)-spiro[4,4]nonane-1,6-diamine (C) ligand which are represented by following stereostructual formula.

[Chemical Formula 7]

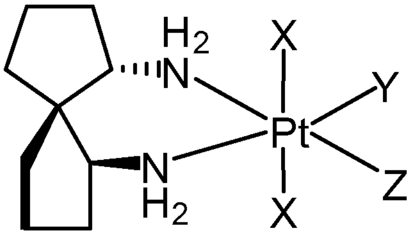

(E)

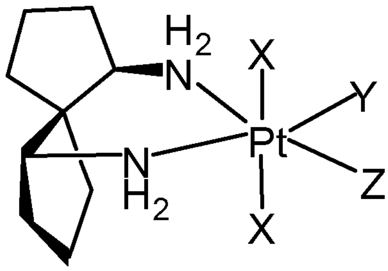

(F)

In the formula, X and Y, Z are same or different, and X and Y, Z represent halogen atoms or monovalent anionic group including acetate group respectively, or Y and Z cooperatively represent the divalent residue which is represented by the formula (b).

[Chemical Formula 8]

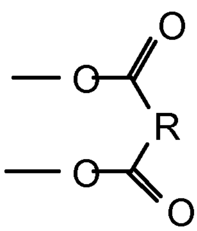

Formula (b)

In the formula, R represents the single bond or represents the straight-chain or branched-chain divalent hydrocarbon residue whose number of carbon atom is 1-6. The hydrocarbon residue may have the unsaturated bond, and the hydrocarbon residue may form the spiro structure.

This invention is dichloromalonato((1S,5S,6S)-spiro[4.4]nonane-1,6-diamine)platinum(IV) which is represented by the following formula (G).

[Chemical Formula 9]

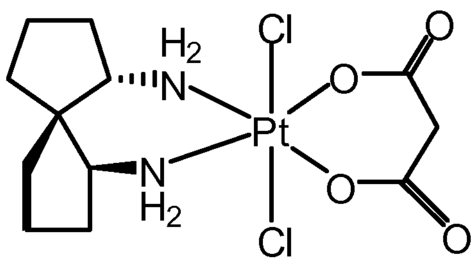

(G)

This invention is a pharmaceutical composition including the platinum(IV) complexes of each above-described formula as an active ingredient.

This invention is a therapeutic agent for malignant tumors including the platinum(IV) complexes of each above-described formula as an active ingredient.

For achieving these objects, the inventors of this application found that the new spiro[4,4]nonane-1,6-diamineplatinum(II) complex which is represented by following general formula (a) and the pharmaceutical composition having this complex as the active ingredient are effective for the therapeutic agent for malignant tumors, and filed the patent application (Japanese patent application NO.2008-225698).

[Chemical Formula 10]

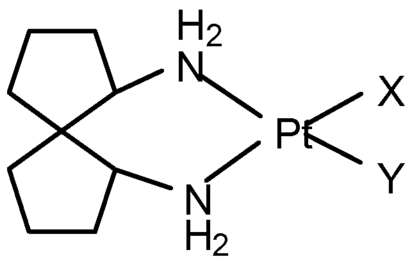

Formula (a)

In the formula, X and Y are same or different, and X and Y represent halogen atoms respectively, or X and Y cooperatively represent the divalent residue which is represented by the formula (b).

[Chemical Formula 11]

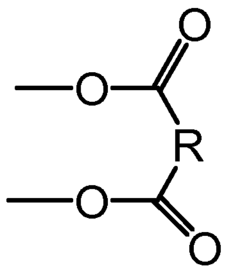

Formula (b)

In the formula, R represents the single bond or represents the straight-chain or branched-chain divalent hydrocarbon residue whose number of carbon atom is 1-6. The hydrocarbon residue may have the unsaturated bond, and the hydrocarbon residue may form the spiro structure.

Besides, it was cleared that platinum(II) complex (H) with (cis,cis-spiro[4,4]nonane-1,6-diamine)oxalato which is especially represented in the following formula (c) had the strong antitumor activity for the malignant tumors, especially the nonsolid tumor such as the human lymphoma cell, and had the feature that the side-effect is reduced relatively.

[Chemical Formula 12]

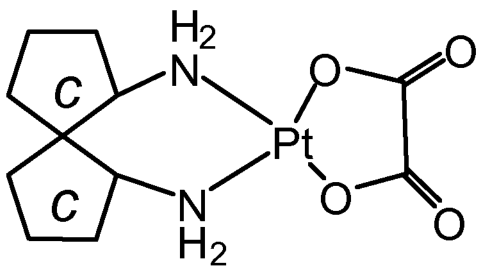

Formula (c)

The cis,cis-spiro[4,4]nonane-1,6-diamine exists as optically active racemate which has two steric structures, that is, (S,S,S) form and (R,R,R) form. The inventors of this application further progressed the invention, investigated these optically active diamine platinum complexes, filed the patent application (Japanese patent application No.2009-155399), and had the patent registration (Patent document No.1).

EFFECT OF THE INVENTION

The new platinum(IV) complexes of this invention has the strong antitumor activity for various malignant tumors

such as human lung cancer cell, human gastric cancer cell, human prostate cancer cell, human malignant melanoma cell, human bladder cancer cell, human lymphoma cell, human leukemia cell, and has the effect compared with the conventional therapeutic agent for the malignant tumor of the platinum complex with smaller doses. Therefore, the side-effect is reduced relatively.

MODE FOR CARRYING OUT THE INVENTION

Hereinafter, the platinum(IV) complexes of this invention and the therapeutic agent for the malignant tumor containing it are explained in connection with the embodiments.

The cis,cis-spiro[4,4]nonane-1,6-diamine (A), the optically active (1S,5S,6S)-spiro[4,4]nonane-1,6-diamine (B), the optically active (1R,5R,6R)-spiro[4,4]nonane-1,6-diamine (C), and the synthesis of the platinum(II) complexes (H), (I), (J) from these have been already explained by the patent of the inventors of this application (Japanese patent application No.2009-155399, International patent application PCT/JP2009/004077).

[Chemical Formula 13]

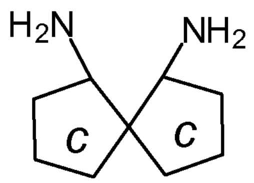

(A)

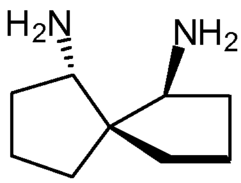

(B)

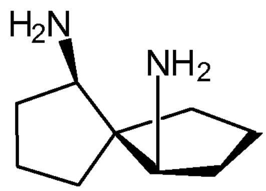

(C)

[Chemical Formula 14]

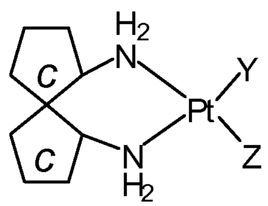

(H)

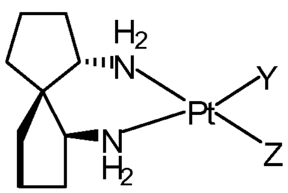

(I)

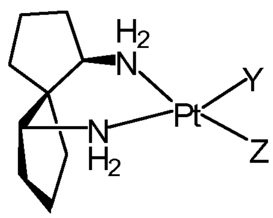

(J)

The platinum(IV) complexes (D), (E), (F) of this invention are obtained easily by applying the heretofore known method, for example the methods which are described in J. Inorg. Biochem., 1996, 61, 291-310, J. Med. Chem., 1997, 40, 112-116 (reaction formula (i) and reaction formula (ii)).

[Chemical Formula 15]

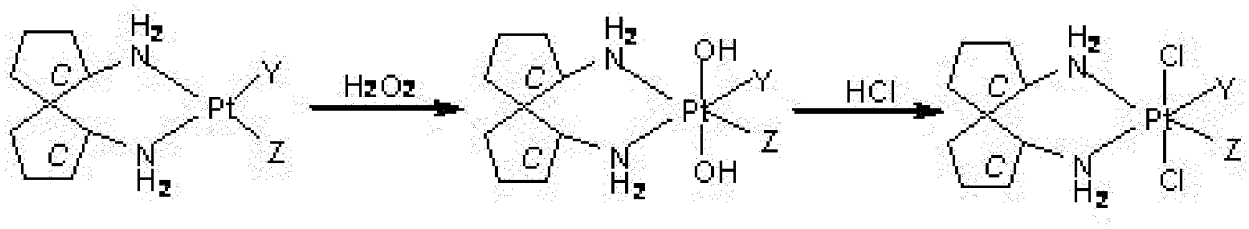


(A) (K) (D)

Reaction Formula (i)

[Chemical Formula 16]

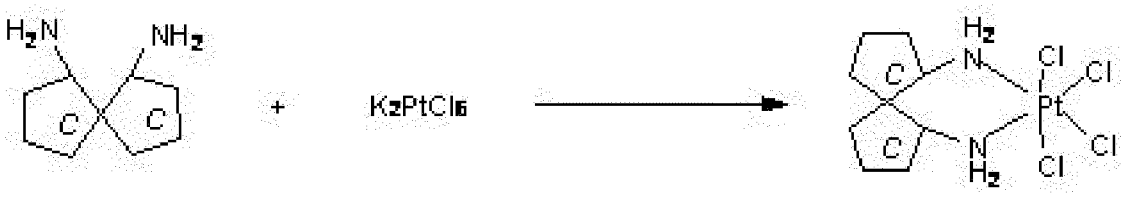

Reaction Formula (ii)

Although there is the case that this complex contains water as an aqueous complex, the aqueous body is also contained in this invention.

It became clear that the complex of this invention blocked the proliferation with low concentration for human lung cancer cell, human gastric cancer cell, human prostate cancer cell, human malignant melanoma cell, human bladder cancer cell, human lymphoma cell, human leukemia cell, and that the complex of this invention showed the strong antitumor action. The complex of this invention is effective for the therapeutic agent for malignant tumors.

When the therapeutic agent which contains the efficacious dose of the platinum complex of this invention is administered in the clinical practice, it is performed by the oral administration or the parenteral administration. The formulation includes such as tablet, sugar-coated tablet, ball, capsule, powdered medicine, lozenge, liquid medicine, suppository, injectable solution, and these are manufactured by blending the excipients which are allowable as medicine. As the excipient, the following one can be exemplified. These are such as lactose, sucrose, glucose, sorbitol, mannitol, potato starch, amylopectin, other various starches, cellulose derivative (for example, carboxymethyl cellulose, hydroxyethyl cellulose), gelatin, calcium stearate, magnesium stearate, polyvinyl alcohol, polyethylene glycol wax, gum arabic, talc, titanium dioxide, vegetable oil such as olive oil, peanut oil or sesame oil, paraffin oil, neutral fat base, ethanol, propylene glycol, saline, sterilized water, glycerin, coloring agent, seasoned formulation, thickener, stabilizer, isotonic agent, buffering agent, and other excipients which are allowable as medicine.

The therapeutic agent of this invention can contain the platinum complexes of this invention of 0.001-85 % by weight, and preferably, can contain the platinum complexes of 0.005-60 % by weight.

The dose of the therapeutic agent of this invention is mainly influenced by the symptom. However, it is 0.005-200 mg per an adult weight per a day, and preferably, it is 0.01-50 mg.

The embodiments are enumerated as follows, and this invention is explained more concretely.

<Embodiment 1>

Malonato((S,S,S)-spiro[4,4]nonane-1,6-diamine)platinum(II) of 0.49g was put in the round bottom flask of 50ml, and the distilled water of 22ml and 30% hydrogen peroxide water of 5.4ml were added, and then these were heated and stirred for 2 hours at 70 degrees centigrade. These were cooled till room temperature and further stirred for 24 hours. The filtration was implemented by celite and the filtered one was washed by water. After concentrating the filtered solution, acetone (200ml) was added and left. Appeared solid was filtrated, washed by acetone and dried. The appeared solid was washed by water, after that by ethanol, further by diethyl ether, and dried. Dihydroxy complexes of malonato((S,S,S) -spiro[4,4]nonane-1,6-diamine)platinum(IV) of 0.48g was obtained. The yield was 93.6%.

This Pt(IV) compound of 0.52g was put in the round bottom flask of 200ml, 0.02N hydrochloric acid of 98ml was added to this, and obtained one was stirred for 4 days in the dark place. The filtration was implemented by celite, the filtered one was washed by water and by methanol, and solvent was distilled away. The residue was solved in methanol, filtered by celite again, and the methanol was distilled away and dried. Dichloro complexes (G) of the malonato((S,S,S)-spiro[4,4]nonane-1,6-diamine)platinum(IV) of 0.33g was obtained.
The yield was 59.4%.

| Elementary analysis | C | H | N | Cl | Pt as $C_{12}H_{20}N_2O_4Cl_2Pt$ |
|---|---|---|---|---|---|
| Calculated value (%) | 27.60 | 3.86 | 5.36 | 13.58 | 37.4 |
| Measured value (%) | 26.95 | 3.92 | 5.16 | 13.11 | 36.7 |

IR(KBr):3177 (N-H), 1645 (C=O), 1371 (C-O) $cm^{-1}$
MS(ESI): m/z 521

From the above results, it was identified that this compound had the chemical structure which was represented by a (compound 1).

[Chemical Formula 17]

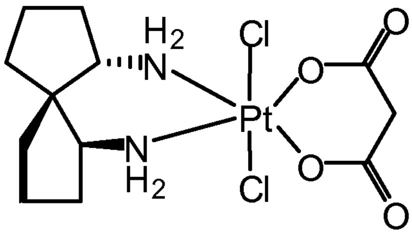

(Compound 1)

<Embodiment 2>

Malonato((R,R,R)-spiro[4,4]nonane-1,6-diamine)platinum(II) of 1.23g was put in the round bottom flask of 50ml, and the distilled water of 18ml and 30% hydrogen peroxide water of 4.4ml were added, and then these were heated and stirred for 2 hours at 70 degrees centigrade. These were cooled till room temperature and further stirred for 24 hours. The filtration was implemented by celite and the filtered one was washed by water. After concentrating the filtered solution, acetone (200ml) was added and left. Appeared solid was filtrated, washed by acetone and dried. Dihydroxy complexes of malonato((R,R,R)-spiro[4,4]nonane-1,6-diamine)platinum(IV) of 1.19g was obtained. The yield was 90.0%.
This Pt(IV) compound of 1.04g was put in the round bottom flask of 300ml, 0.02N hydrochloric acid of 204ml was added to this, and obtained one was stirred for 4 days in the dark place. The filtration was implemented by celite, the filtered one was washed by water and by methanol, and solvent was distilled away. The residue was solved in methanol, filtered by celite again, and the methanol was distilled away and dried. An optical isomer of Dichloro complexes (G) of the malonato((R,R,R)-spiro[4,4]nonane-1,6-diamine)platinum(IV) of 0.84g was obtained. The yield was 74.8%.
The result of IR measurement corresponded to the platinum(IV) complex of the embodiment 1, and it was identified as a (compound 2).

[Chemical Formula 18]

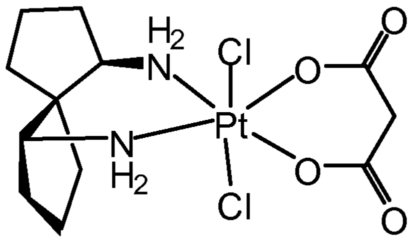

(Compound 2)

<Embodiment 3>

Oxalato((S,S,S)-spiro[4.4]nonane-1,6-diamine)platinum(II) of 1.07g was put in the round bottom flask of 50ml, and the distilled water of 50ml and 30% hydrogen peroxide water of 12.4ml were added, and then these were heated and stirred for 2 hours at 70 degrees centigrade. These were cooled till room temperature and further stirred for 2 days. The filtration was implemented by celite and the filtered one was washed by water. After concentrating the filtered solution, acetone (200ml) was added and left. Appeared solid was filtrated, washed by acetone and dried. The appeared solid was washed by water, after that ethanol, further diethyl ether, and dried. Dihydroxy complexes of an oxalato((S,S,S)-spiro[4.4]nonane-1,6-diamine)platinum(IV)of 1.15g was obtained. The yield was 99.6%.

This Pt(IV) compound of 0.88g was put in the round bottom flask of 300ml, the distilled water of 165ml and 1.2N hydrochloric acid of 2.85ml were added to this, and obtained one was stirred for 3 days in the dark place. The filtration was implemented by celite, the filtered one was washed by water and by methanol, and solvent was distilled away. The residue was solved in methanol, filtered by celite again, and the methanol was distilled away and dried. Dichloro complexes of the oxalato((S,S,S) -spiro[4.4]nonane-1,6-diamine)platinum(IV) of 0.79g was obtained. The yield was 83.1%.

| Elementary analysis | C | H | N | Cl | Pt as $C_{11}H_{18}N_2O_4Cl_2Pt$ |
|---|---|---|---|---|---|
| Calculated value (%) | 25.99 | 3.57 | 5.51 | 13.95 | 38.4 |
| Measured value (%) | 25.55 | 3.65 | 5.26 | 13.35 | 37.9 |

IR(KBr):3175 (N-H), 1715 (C=O), 1357 (C-O) $cm^{-1}$
MS(ESI): m/z 507

From the above results, it was identified that this compound had the chemical structure which was represented by a (compound 3).

[Chemical Formula 19]

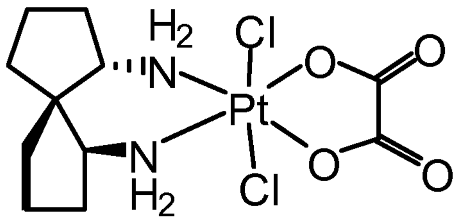

(Compound 3)

<Embodiment 4>

Oxalato((R,R,R)-spiro[4.4]nonane-1,6-diamine)platinum(II) of 1.06g was put in the round bottom flask of 50ml, and the distilled water of 49ml and 30% hydrogen peroxide water of 12.8ml were added, and then these were heated and stirred for 2 hours at 70 degrees centigrade. These were cooled till room temperature and further stirred for 3 days. The filtration was implemented by celite and the filtered one was washed by water. After concentrating the filtered solution, acetone (250ml) was added and left. Appeared solid was filtrated, washed by acetone and dried. Dihydroxy complexes of an oxalato((R,R,R) -spiro[4.4]nonane-1,6-diamine)platinum(IV) of 1.11g was obtained. The yield was 97.3%.

This Pt(IV) compound of 0.92g was put in the round bottom flask of 300ml, the distilled water of 190ml and 1.2N hydrochloric acid of 3.3ml were added to this, and obtained one was stirred for 3 days in the dark place. The filtration was implemented by celite, the filtered one was washed by water and by methanol, and solvent was distilled away. The residue was solved in methanol, filtered by celite again, and the methanol was distilled away and dried. Dichloro complexes of the oxalato((R,R,R) -spiro[4.4]nonane-1,6-diamine)platinum(IV) of 0.92g was obtained. The yield was 92.4%.

The result of IR measurement corresponded to the platinum(IV) complex of the embodiment 3, and it was identified as a (compound 4).

[Chemical Formula 20]

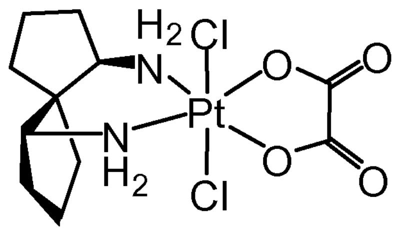

(Compound 4)

<Embodiment 5>

Potassium hexachloroplatinate(IV) of 0.49g, sodium chloride of 0.24g and the distilled water of 60ml were put in the round bottom flask of 100ml and solved. The water solution of 10ml of the (S,S,S)-spiro[4,4]nonane-1,6-diamine of 0.16g was added to this solution. This was roiled gradually and the solid appeared. This was stirred for 24 hours in the dark place. The filtration was implemented and the filtered one was washed by water and dried. Tetrachloro((S,S,S)-spiro[4.4]nonane-1,6-diamine)platinum(IV) of 0.22g was obtained. The yield was 43.6%.

| Elementary analysis | C | H | N | Cl | Pt as $C_9H_{18}N_2Cl_4Pt$ |
|---|---|---|---|---|---|
| Calculated value (%) | 22.01 | 3.69 | 5.70 | 28.87 | 39.7 |
| Measured value (%) | 21.54 | 3.58 | 5.36 | 24.88 | 39.2 |

IR(KBr):3227 (N-H) $cm^{-1}$
MS(ESI): m/z 490

From the above results, it was identified that this compound had the chemical structure which was represented by a (compound 5).

[Chemical Formula 21]

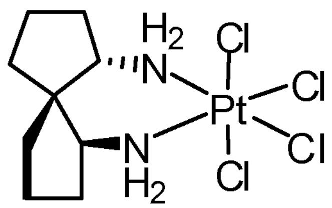

(Compound 5)

<Embodiment 6>

As well as the embodiment 5, tetrachloro((R,R,R)-spiro[4.4]nonane-1,6-diamine)platinum(IV) of 0.27g was obtained from the potassium hexachloroplatinate(IV) and (R,R,R)-spiro[4,4]nonane-1,6-diamine of 0.16g. The yield was 55.0%.
From the result of the IR measurement, this compound corresponded to the platinum(IV) complex of the Embodiment 5, and it was identified that this compound was a (compound 6).

[Chemical Formula 22]

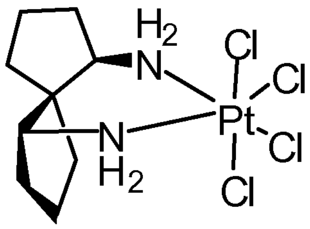

(Compound 6)

<Embodiment 7> Medicine effect test

The test solution was prepared by dissolving the (compound 1) into the DMSO (dimethylsulfoxide) with the concentration of 8mg/ml.

The test was implemented by using the human lung cancer cell (LU99), two kind of the human gastric cancer cell (KATO III, MKN-45), the human prostate cancer cell (DU145), the human malignant melanoma cell (G-361), the human bladder cancer cell (T24), two kind of the human lymphoma cell (U937, Jurkat E6.1) and the human leukemia cell (HL60) as the cancer cell.

These cells were suspended in the each culture medium that the serum of 10% was added, and were dispensed in 96-well plate. And then, these were cultured in 5%$CO_2$ at 37 degrees centigrade during a night. The test solution was prepared in the culture medium to the various concentrations, and was dispensed into the plate that the cells were set preliminarily. And further, these were cultured in 5%$CO_2$ at 37 degrees centigrade for three days.

The cell proliferation after the medicine addition was measured on the 3rd day from the 1st day after the medicine addition by the MTS method (Kit for cell proliferation test manufactured by Promega Company, Cell Titer 96 Aqueous One Solution Cell Proliferation Assay).

The inhibition rate (%) of the cell proliferation was obtained from the measured MTS value by the following formula.

$$\text{Inhibition rate (\%)} = (1\text{-MTS value of medicine addition group/MTS value of medicine non-addition group}) \times 100$$

Because the value which was obtained by the above-mentioned formula represents the inhibition rate of the cell proliferation, the higher the numerical value is, the higher the medicine effect is. It is deemed that the one whose value is 50% or more has the medicine effect. The result of the kind of the cell which has the high effect is represented below.

[Table 1]

| Tab.1 Inhibition rate of cell proliferation after 3 days from medicine addition (%) | | | | | | | |
|---|---|---|---|---|---|---|---|
| | | (Compound 1) | | | Oxaliplatin | | |
| Concentration of medicinal solution ($\mu$g/ml) | | 5 | 10 | 20 | 5 | 10 | 20 |
| Human gastric cancer cell | KATO III | 53 | 75 | 88 | 58 | 75 | 87 |
| Human lymphoma cell | U937 | 98 | 102 | 101 | 67 | 90 | 99 |
| | Jurkat E6.1 | 92 | 97 | 100 | 90 | 94 | 97 |
| Human leukemia cell | HL60 | 91 | 97 | 99 | 73 | 86 | 93 |

As for the compound 1, it became clear that the medicine effect of the (compound 1) was stronger in the human lymphoma cell and the human leukemia cell as compared with the oxaliplatin which was conventional anticancer agent. Especially, the inhibition rate of the cell proliferation in the low concentration (5$\mu$g/ml) is high, and the compound 1 was effective with a small quantity than the oxaliplatin.

<Embodiment 8> Medicine effect test

The (compound 2) was prepared as the test solution, and the inhibition rate of the cell proliferation was measured by the method similar to the embodiment 7. The result of the kind of the cell which has the high effect is represented below.

[Table 2]

| Tab.2 Inhibition rate of cell proliferation after 3 days from medicine addition (%) | | | | | | | |
|---|---|---|---|---|---|---|---|
| | | (Compound 2) | | | Oxaliplatin | | |
| Concentration of medicinal solution ($\mu$g/ml) | | 5 | 10 | 20 | 5 | 10 | 20 |
| Human gastric cancer cell | KATO III | 78 | 90 | 82 | 58 | 75 | 87 |
| Human prostate cancer cell | DU145 | 51 | 76 | 84 | 46 | 71 | 81 |
| Human bladder cancer cell | T24 | 52 | 77 | 87 | 38 | 43 | 48 |
| Human lymphoma cell | U937 | 100 | 100 | 100 | 67 | 90 | 99 |

As for the compound 2, it became clear that the medicine effect of the (compound 2) was stronger in the human gastric cancer cell, the human bladder cancer cell and the human lymphoma cell as compared with the oxaliplatin which was conventional anticancer agent. Especially, the inhibition rate of the cell proliferation in the low concentration (5 and 10$\mu$g/ml) is high, and the compound 2 was effective with a small quantity than the oxaliplatin.

<Embodiment 9> Medicine effect test

The (compound 5) was prepared as the test solution, and the inhibition rate of the cell proliferation was measured by the method similar to the embodiment 7. The result of the kind of the cell which has the high effect is represented below.

[Table 3]

| Tab.3 Inhibition rate of cell proliferation after 3 days from medicine addition (%) | | | | | | | |
|---|---|---|---|---|---|---|---|
| | | (Compound 5) | | | Oxaliplatin | | |
| Concentration of medicinal solution ($\mu$g/ml) | | 5 | 10 | 20 | 5 | 10 | 20 |
| Human lung cancer cell | LU99 | 65 | 84 | 91 | 38 | 40 | 49 |
| Human gastric cancer cell | MKN-45 | 90 | 93 | 87 | 73 | 82 | 87 |
| Human malignant melanoma cell | G-361 | 83 | 95 | 98 | 50 | 59 | 74 |
| Human bladder cancer cell | T24 | 79 | 86 | 80 | 38 | 43 | 48 |

As for the compound 5, it became clear that the medicine effect of the (compound 5) was stronger in the human lung cancer cell, the human gastric cancer cell, the human malignant melanoma cell and the human bladder cancer cell as compared with the oxaliplatin which was conventional anticancer agent. Especially, the inhibition rate of the cell proliferation in the low concentration (5 and 10$\mu$g/ml) is high, and the compound 5 was effective with a small quantity than the oxaliplatin.

<Embodiment 10> Medicine effect test

The (compound 6) was prepared as the test solution, and the inhibition rate of the cell proliferation was measured by the method similar to the embodiment 7. The result of the kind of the cell which has the high effect is represented below.

[Table 4]

| Tab.4 Inhibition rate of cell proliferation after 3 days from medicine addition (%) | | | | | | | |
|---|---|---|---|---|---|---|---|
| | | (Compound 6) | | | Oxaliplatin | | |
| Concentration of medicinal solution ($\mu$g/ml) | | 5 | 10 | 20 | 5 | 10 | 20 |
| Human lung cancer cell | LU99 | 89 | 96 | 98 | 38 | 40 | 49 |

(continued)

| Tab.4 Inhibition rate of cell proliferation after 3 days from medicine addition (%) | | | | | | | | |
|---|---|---|---|---|---|---|---|---|
| | | (Compound 6) | | | Oxaliplatin | | |
| Concentration of medicinal solution ($\mu$g/ml) | | 5 | 10 | 20 | 5 | 10 | 20 |
| Human gastric cancer cell | MKN-45 | 88 | 80 | 76 | 73 | 82 | 87 |
| Human malignant melanoma cell | G-361 | 89 | 96 | 98 | 50 | 59 | 74 |
| Human bladder cancer cell | T24 | 78 | 81 | 81 | 38 | 43 | 48 |

As for the compound 6, it became clear that the medicine effect of the (compound 6) was stronger in the human lung cancer cell, the human malignant melanoma cell and the human bladder cancer cell as compared with the oxaliplatin which was conventional anticancer agent. Especially, the inhibition rate of the cell proliferation in the low concentration (5 and 10$\mu$g/ml) is high, and the compound 6 was effective with a small quantity than the oxaliplatin.

From the above results of the embodiment 7-10, the new platinum(IV) complexes of this invention has the strong antitumor activity for various malignant tumors such as human lung cancer cell, human gastric cancer cell, human prostate cancer cell, human malignant melanoma cell, human bladder cancer cell, human lymphoma cell, human leukemia cell, and has the effect compared with the conventional therapeutic agent for the malignant tumor of the platinum complex with smaller doses. Therefore, the side-effect is reduced relatively.

INDUSTRIAL APPLICABILITY

As described above, the platinum complex of this invention has the strong antitumor activity, and is efficacious as the therapeutic agent for the malignant tumor.

**Claims**

1. Platinum(IV) complexes, **characterized by** comprising:

cis,cis-spiro[4,4]nonane-1,6-diamine (A) ligand represented by a following stereostructual formula.

[Chemical Formula 1]

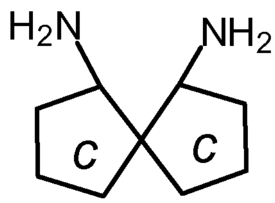

(A)

2. The platinum(IV) complexes, according to claim 1, **characterized by** comprising:

Optically active (S,S,S)-spiro[4,4]nonane-1,6-diamine (B) ligand and optically active (R,R,R)-spiro[4,4]nonane-1,6-diamine (C) ligand represented by following stereostructual formulas.

[Chemical Formula 2]

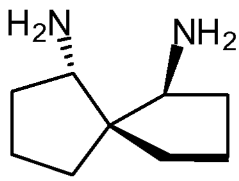

(B)

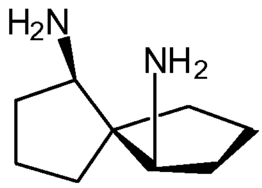

(C)

**3.** The platinum(IV) complexes, according to claim 1, **characterized by** comprising:

cis,cis-spiro[4,4]nonane-1,6-diamine (A) ligand represented by a following general formula (D).

[Chemical Formula 3]

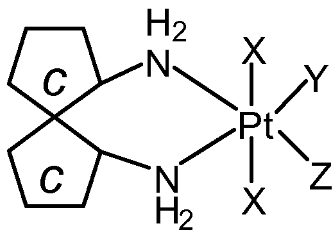

(D)

(In the formula, X and Y, Z are same or different, and X and Y, Z represent halogen atoms or monovalent anionic group including acetate group respectively, or Y and Z cooperatively represent the divalent residue which is represented by the formula (b).)

[Chemical Formula 4]

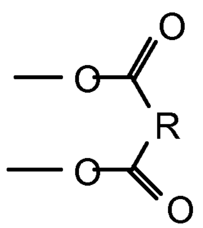

Formula (b)

(In the formula, R represents the single bond or represents the straight-chain or branched-chain divalent hydrocarbon residue whose number of carbon atom is 1-6. The hydrocarbon residue may have the unsaturated bond, and the hydrocarbon residue may form the spiro structure.)

**4.** The platinum(IV) complexes, according to claim 2, **characterized by** comprising:

Optically active (S,S,S)-spiro[4,4]nonane-1,6-diamine (B) ligand and optically active (R,R,R)-spiro[4,4]nonane-1,6-diamine (C) ligand represented by following stereostructual formulas (E) and (F).

[Chemical Formula 5]

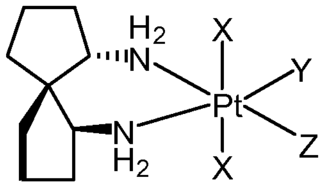

(E)

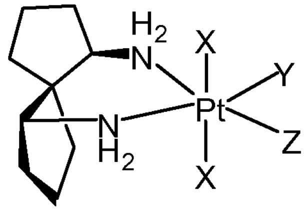

(F)

(In the formula, X and Y, Z are same or different, and X and Y, Z represent halogen atoms or monovalent anionic group including acetate group respectively, or Y and Z cooperatively represent the divalent residue which is represented by the formula (b).)

[Chemical Formula 6]

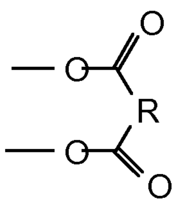

Formula (b)

(In the formula, R represents the single bond or represents the straight-chain or branched-chain divalent hydrocarbon residue whose number of carbon atom is 1-6. The hydrocarbon residue may have the unsaturated bond, and the hydrocarbon residue may form the spiro structure.)

**5.** The platinum(IV) complexes, according to claim 3 and claim 4, **characterized in that**:

X are chlorine atoms.

**6.** Dichloromalonatoplatinum(IV) complexes according to any one of claims of claim 1 to claim 4, **characterized by** comprising:

the ((S,S,S)-spiro[4,4]nonane-1,6-diamine) ligand represented by a following formula (G), wherein, X are chlorine atoms.

[Chemical Formula 7]

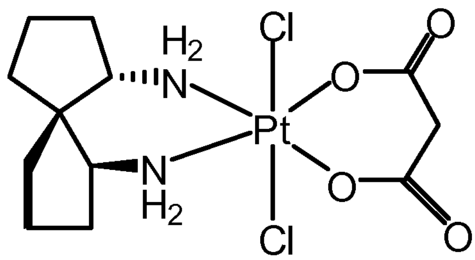

(G)

**7.** A pharmaceutical composition, **characterized in that**:

the platinum(IV) complexes described in any one of claims of claim 1 to claim 6 is contained as an active ingredient.

**8.** A therapeutic agent for a malignant tumor, **characterized in that**:

the platinum(IV) complexes described in any one of claims of claim 1 to claim 6 is contained as an active ingredient.

**INTERNATIONAL SEARCH REPORT**

International application No.
PCT/JP2012/067471

A. CLASSIFICATION OF SUBJECT MATTER
*C07C211/38*(2006.01)i, *A61K31/282*(2006.01)i, *A61P35/00*(2006.01)i, *C07C55/08*(2006.01)i, *C07F15/00*(2006.01)n

According to International Patent Classification (IPC) or to both national classification and IPC

B. FIELDS SEARCHED

Minimum documentation searched (classification system followed by classification symbols)
C07C211/38, A61K31/282, A61P35/00, C07C55/08, C07F15/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | WO 2010/026711 A1 (Unitech Co., Ltd.), 11 March 2010 (11.03.2010), claims 1 to 15; paragraphs [0001], [0008]; examples 1 to 8 & JP 2010-83867 A & EP 2325163 A1 & CA 2734125 A & CN 102143936 A & KR 10-2011-0055594 A | 1-8 |
| A | WO 2010/082503 A1 (Unitech Co., Ltd.), 22 July 2010 (22.07.2010), claims 1 to 4; paragraphs [0001], [0008], [0009]; examples 1, 2 & JP 2010-163407 A | 1-8 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 September, 2012 (28.09.12) | 09 October, 2012 (09.10.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2012/067471

C (Continuation). DOCUMENTS CONSIDERED TO BE RELEVANT

| Category* | Citation of document, with indication, where appropriate, of the relevant passages | Relevant to claim No. |
|---|---|---|
| A | WO 2005/007662 A2 (JOHNSON MATTHEY PLC), 27 January 2005 (27.01.2005), example 14<br>& US 2007/0066850 A1 & GB 316439 D<br>& EP 1651657 A1 & DE 602004008478 D<br>& AT 370959 T | 1-8 |
| A | JP 2-108693 A (Nederlandse Organisatie Voor Toegepast-Natuurwetenschappelijk Onderzoek Tno), 20 April 1990 (20.04.1990), claim 1; page 3, lower left column, lines 9 to 13; examples Ia, Ib, Ic; tables B, C, D<br>& US 5028727 A & EP 357108 A2<br>& CN 1040591 A | 1-8 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4664424 B
- US 4140707 A
- JP 2008225698 A
- JP 2009155399 A
- JP 2009004077 W


### Non-patent literature cited in the description


- *Nature,* 1969, vol. 222, 385-386
- *Cancer Treat Reviews,* 1985, vol. 12, 21-33
- *Cancer Letters,* 1985, vol. 27, 135-143
- *J. Inorg. Biochem.,* 1996, vol. 61, 291-310
- *J. Med. Chem.,* 1997, vol. 40, 112-116